Europäisches Patentamt

**European Patent Office**

**Office européen des brevets**

(11) Publication number: **0 123 265**
**A1**

(12) **EUROPEAN PATENT APPLICATION**

(21) Application number: **84104371.4**

(22) Date of filing: **18.04.84**

(51) Int. Cl.³: **G 01 N 33/54**
**G 01 N 33/58, C 12 Q 1/38**

(30) Priority: **18.04.83 US 486016**

(43) Date of publication of application:
**31.10.84 Bulletin 84/44**

(84) Designated Contracting States:
**AT BE CH DE FR GB IT LI LU NL SE**

(71) Applicant: **E.I. DU PONT DE NEMOURS AND COMPANY**
**1007 Market Street**
**Wilmington Delaware 19898(US)**

(72) Inventor: **Hall, James Edward, Jr.**
**2600 W. 7th Street**
**Wilmington Delaware 19805(US)**

(72) Inventor: **Hargreaves, William Rudolph**
**1400 Forrest Road**
**Wilmington Delaware 19810(US)**

(74) Representative: **Abitz, Walter, Dr.-Ing. et al,**
**Abitz, Morf, Gritschneder, Freiherr von Wittgenstein**
**Postfach 86 01 09**
**D-8000 München 86(DE)**

(54) **Zymogen activation, cascade amplification immunoassay.**

(57) Sensitive immunochemical analysis methods, and novel materials to practice the methods, are provided for the quantitative determination of analytes in liquid medium. Conjugates of an analyte and a zymogen or activator; activator or zymogen specific for the conjugated labeling substance; binding agent specific for the analyte; and in some cases other zymogens, are mixed with the liquid medium suspected of containing the analyte to produce zymogen-derived-enzyme which can either directly generate detectable marker material or trigger an enzyme cascade activation sequence which amplifies the production of a detectable marker material. The concentration or rate of production of the marker material is correlated with the amount of analyte initially present. The method can be adapted to automated techniques.

*Fig. 4*

CASCADE AMPLIFIED DIGOXIN ASSAY

TITLE

ZYMOGEN ACTIVATION, CASCADE
AMPLIFICATION IMMUNOASSAY

TECHNICAL FIELD

This invention relates to highly sensitive and rapid methods of immunochemical analysis for the quantitative determination of the amount of a specific analyte in liquid medium, and reagent means to practice the method, in which an immunoassay technique utilizing initially inactive conjugate containing analyte or binding agent and a zymogen or a zymogen activator is chemically coupled with a zymogen-to-enzyme activation cascade to effect a large multiplication in measurement sensitivity.

BACKGROUND OF THE INVENTION

Clinical laboratory chemical diagnostic tests are an important component of health care delivery. The utilization of these tests by physicians to monitor drug levels where only a narrow therapeutic range exists, to guide decisions on treatment and surgical options, and to screen patients for the early detection of disease has rapidly increased the number of tests performed annually. With almost 6 billion tests performed in 1976 and 12.2 billion estimated as being performed in 1986 [Luning Prak Associates Survey, 1980], speed, accuracy, and cost control are important objectives. The desire to measure such analytes as drugs, hormones, and metabolites at micromolar ($\mu M$) to picomolar (pM) levels in complex body fluid matrices has led to the development of sophisticated test methodology which can be implemented by automated techniques to maintain a reasonable cost.

Broadly applicable, accurate screening assay technologies are therefore needed to monitor the presence and quantity of biological materials. Various methods have been utilized in the past including liquid and gas chromatography, mass spectrometry, and numerous bioassay techniques. These methods are time consuming and not easily applied in large-scale, automated screening programs.

In recent years, a number of enzyme immunoassay techniques have been developed to take advantage of the specificity of antibody reactions while avoiding the complicating features of radiochemical labeling. These assays frequently employ antibody specific for the analyte of interest to modulate enzymatic activity directly, using an analyte-labeled enzyme, or indirectly by reacting with analyte-labeled cofactor required by the enzyme for activity. Such systems can be adapted for quantifying medically important materials including cardiac glycosides, antibiotics, therapeutic drugs, hormones, and vitamins. In addition, such methods of analysis are useful for toxins, food and packaging additives, and environmental pollutants at low concentration.

Typically, competitive binding immunoassay techniques involve a conjugate of a labeling substance attached to an immunochemical binding component which participates in a reaction with the other component of the immunological pair to produce two species of the conjugate, namely a bound complex and a free species. The relative amounts of conjugate distributed in the bound and free products are a function of the concentration of the analyte whose presence and amount are being measured.

U.S. Patent 3,817,837, issued June 18, 1974 to Rubenstein and Ullman, discloses an immunological assay method for analytes in solution based upon the inhibition of enzymatic activity of an analyte-labeled enzyme by antibody to the analyte. The formation of a reaction system consisting of free analyte in the test medium, analyte-labeled enzyme, and antibody specific for the analyte results in a competition for the antibody. Since the reaction of antibody with analyte-labeled enzyme modifies the activity of the enzyme, the surviving enzymatic activity of the solution is related to the amount of analyte present in the test medium. Such assay systems which incorporate active enzyme are in general somewhat unstable and are sensitive to thermal and hydrolytic processes which may lead to enzyme denaturation necessitating frequent recalibration of the standard curve used to determine analyte concentration. In addition, since analyte may be attached to enzyme at sites where antibody binding does not inhibit enzyme activity, as many as 30-40 analyte molecules per enzyme molecule may have to be covalently attached to the active enzyme to insure modulation by antibody binding. This heavy analyte labeling can decrease the sensitivity of the assay.

Another approach to biological assay of analytes in solution is disclosed by U.S. Patent 4,238,565 issued December 9, 1980 to Hornby et al. In this method, an obligatory bound cofactor is stripped from an active holoenzyme using extreme pH to create an inactive apoenzyme. The analyte to be measured is chemically bonded to cofactor and antibody to the analyte is prepared. The formation of a reaction system consisting of the test medium suspected of containing analyte, the apoenzyme, the antibody, and the analyte-labeled cofactor results in a competition for the antibody. Unreacted analyte-labeled cofactor then combines with apoenzyme to yield an active holoenzyme which catalyzes the production of a photometrically detectable material. While the signal measured is directly proportional to the concentration of analyte present in the test medium, such systems typically require slow conformational rearrangements to establish maximal enzyme activity, requiring at least 20-120 minutes incubation time. Apoenzymes are generally less stable than their corresponding holoenzymes. Special stabilizing techniques may be required to perform such assays at 37°C, the standard temperature of most enzyme assays and automated clinical systems. Cofactors are also typically small molecules and it is difficult to modify them without significantly altering their activity.

The above described assays are said to be homogeneous because no separation step is required in the measurement process. Where the conjugate in the bound and the free species is essentially indistinguishable by the means used to measure the labeling substance, the two species must be physically separated. This type of assay is referred to as heterogeneous.

U.S. Patent 4,009,005, issued February 2, 1977 to Johnson, discloses a competitive heterogeneous immunoassay in which sample analyte and a limited amount of labeled analyte compete for binding to a limited amount of immobilized anti-analyte antibody. The labeling substance is typically a radioisotope. Either the bound or the free fraction can be quantified.

A noncompetitive heterogeneous immunoassay is disclosed in U.S. Patent 3,654,090, issued April 4, 1972 to Schuurs et al. Sample analyte is first reacted with an excess of enzyme-labeled anti-analyte antibody and then analyte-bound anti-analyte antibody is separated from unbound anti-analyte antibody by contacting the mixture with immobilized analyte. Usually, the fraction which does not bind to the solid phase is quantified. It is suggested that the enzymic labeling substance can convert a proenzyme into an enzyme.

U.S. Re. 31,006, reissued August 3, 1982 to Schuurs et al., discloses a noncompetitive heterogeneous immunoassay of the sandwich type. Sample analyte is first allowed to react with excess immobilized anti-analyte antibody, followed by reaction of the solid phase with an excess of enzyme-labeled anti-analyte antibody. The enzymatic activity of either the solid or the liquid phase is then quantified.

U.S. Patent 4,434,236 describes a heterogeneous immunoassay in which excess labeled anti-analyte antibody immunochemically bound to immobilized analyte-analog is displaced in an amount proportional to the amount of analyte present in the sample. The displaced label is then quantified.

## SUMMARY OF THE INVENTION

At this time there exists a clear need for sensitive biochemical assay techniques which combine specificity and reagent stability to measure analyte concentrations less than 10 nanomolar (nM) at incubation times of 1-10 minutes. These systems should be flexible to enable adaptation to automated clinical instruments.

The instant invention provides novel homogeneous immunoassay techniques and reagents to measure the amount of analyte present in liquid media rapidly with high sensitivity. By utilizing analyte-dependent activation of initially inactive zymogen, there is minimum background "noise" present in the assay. Zymogens are typically extremely stable under a wide range of pH, temperature, and ionic strength, thereby preserving reagent activity and minimizing the need to recalibrate the standard curves used to calculate analyte amount. The use of zymogen activation also provides a unique opportunity to couple the active zymogen-derived-enzyme to a zymogen-to-enzyme cascade reaction sequence to obtain multiple stages of amplification in producing detectable marker material used to quantify analyte amount. This cascade sequence results in a significant increase in sensitivity. Specifically, one aspect of this invention involves a conjugate of an analyte and a labeling substance selected from the group consisting of zymogen, enzymatic activator, and nonenzymatic activator wherein at least one analyte derivative molecule is attached per molecule of labeling substance. The homogeneous competitive measurement aspect of this invention relates to a method for determining the amount of an analyte in liquid medium comprising the steps of:

  A)  forming a reaction system by contacting said liquid medium with

  (1)  conjugate of an analyte and a zymogen;

  (2)  analyte specific binding agent;

  (3)  activator specific for the conversion of said conjugate of an analyte and a zymogen to zymogen-derived-enzyme;

  (4)  substrate reagent convertible by said zymogen-derived-enzyme to a detectable marker material

in proportions such that the conversion of uncombined conjugate of an analyte and a zymogen by said activator to said zymogen-derived-enzyme results in the production of said marker material; and

B) measuring the concentration of said marker material produced which is related to the amount of analyte initially present in said liquid medium.

A heterogeneous competitive measurement aspect of this invention relates to a method for determining the amount of an analyte in liquid medium comprising the steps of:

A) forming a reaction system by contacting said liquid medium with

    (1) conjugate of an analyte and a labeling substance selected from the group consisting of zymogen, enzymatic activator, and nonenzymatic activator;

    (2) insolubilized binding agent:

B) allowing the immunochemical reaction to proceed to form free and bound conjugate;

C) separating said conjugate bound to said insolubilized binding agent from said conjugate remaining free in solution;

D) contacting and reacting said conjugate of an analyte and a labeling substance free in solution with a corresponding bioreactive substance selected from the group consisting of activator and zymogen, and with substrate reagent convertible by the resulting zymogen-derived-enzyme to a detectable marker material; and

E) measuring the amount of said marker material produced which is related to the amount of analyte initially present in said liquid medium;

with the proviso that when said labeling substance is zymogen, said corresponding bioreactive substance is activator; when said labeling substance is enzymatic activator, said corresponding bioreactive substance is zymogen; and when said labeling substance is nonenzymatic activator, said corresponding bioreactive substance is zymogen.

Another aspect of this invention involves a conjugate of a specific binding agent and a labeling substance selected from the group consisting of zymogen, enzymatic activator, and nonenzymatic activator wherein at least one molecule of said binding agent is attached per molecule of labeling substance.

A heterogeneous noncompetitive measurement aspect of this invention relates to a method for determining the amount of an analyte in liquid medium comprising the steps of:

A) forming a reaction system by contacting said liquid medium with

    (1) conjugate of a specific binding agent and a labeling substance selected from the group consisting of zymogen, enzymatic activator, and nonenzymatic activator;

    (2) insolubilized analyte;

B) allowing the immunochemical reaction to proceed to form free and bound conjugate;

C) separating said conjugate bound to said insolubilized analyte from said conjugate remaining free in solution;

D) contacting and reacting said conjugate of a specific binding agent and a labeling substance free in solution with a corresponding bioreactive substance selected from the group consisting of activator and zymogen, and with substrate reagent convertible by the resulting zymogen-derived-enzyme to a detectable marker material; and

E) measuring the amount of said marker material produced which is related to the amount of analyte initially present in said liquid medium;

with the proviso that when said labeling substance is zymogen, said corresponding bioreactive substance is activator; when said labeling substance is enzymatic activator, said corresponding bioreactive substance is zymogen; and when said labeling substance is nonenzymatic activator, said corresponding bioreactive substance is zymogen.

Finally, a heterogeneous noncompetitive "sandwich" measurement technique aspect of this invention relates to a method for detemining the amount of an analyte in liquid medium comprising the steps of:

A) forming a reaction matrix by contacting said liquid medium with insolubilized binding agent to produce bound insolubilized analyte;

B) contacting said reaction matrix formed in step A) with conjugate of a specific binding agent and a labeling substance selected from the group consisting of zymogen, enzymatic activator, and nonenzymatic activator;

C) rinsing said reaction matrix of step B) with solvent to remove unbound reagent;

D) contacting and reacting said reaction matrix of step C) with a corresponding bioreactive substance selected from the group consisting of activator and zymogen, and with substrate reagent convertible by the resulting zymogen-derived-enzyme to a detectable marker material; and

E) measuring the amount of said marker material produced which is related to the amount of analyte initially present in said liquid medium;

with the proviso that when said labeling substance is zymogen, said corresponding bioreactive substance is activator; when said labeling substance is enzymatic activator, said corresponding bioreative substance is zymogen; and when said labeling substance is nonenzymatic activator, said corresponding bioreactive substance is zymogen.

## BRIEF DESCRIPTION OF THE DRAWINGS

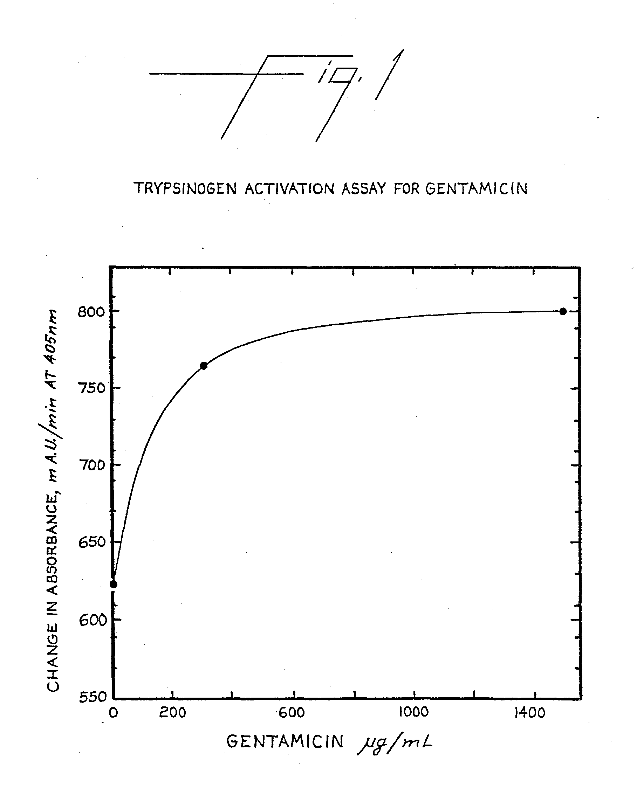

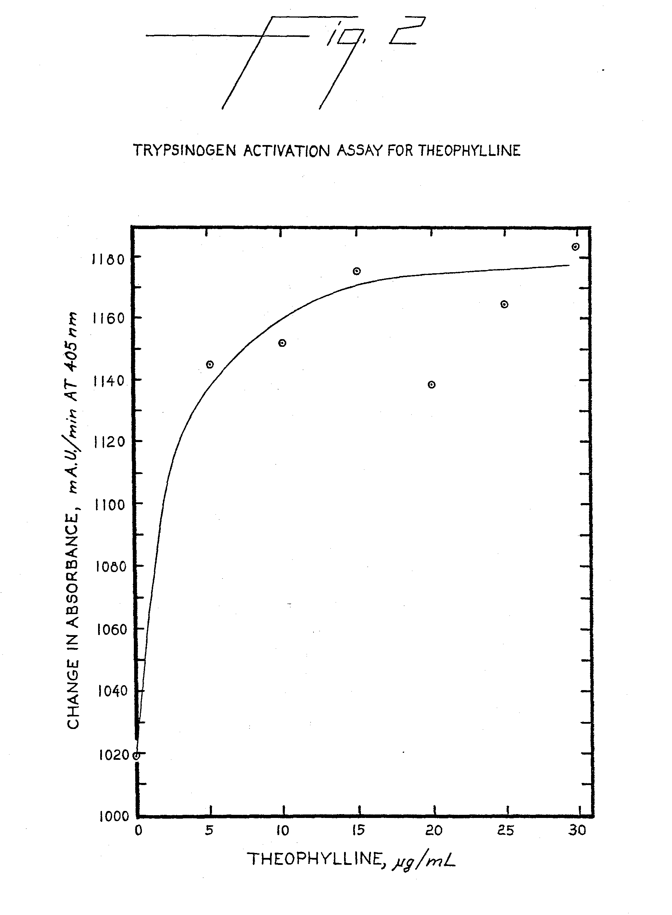

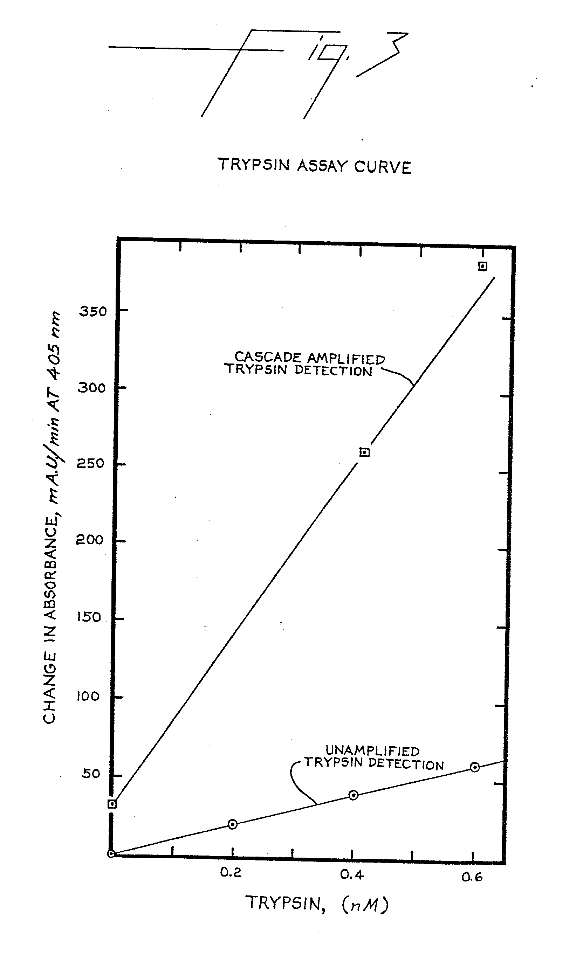

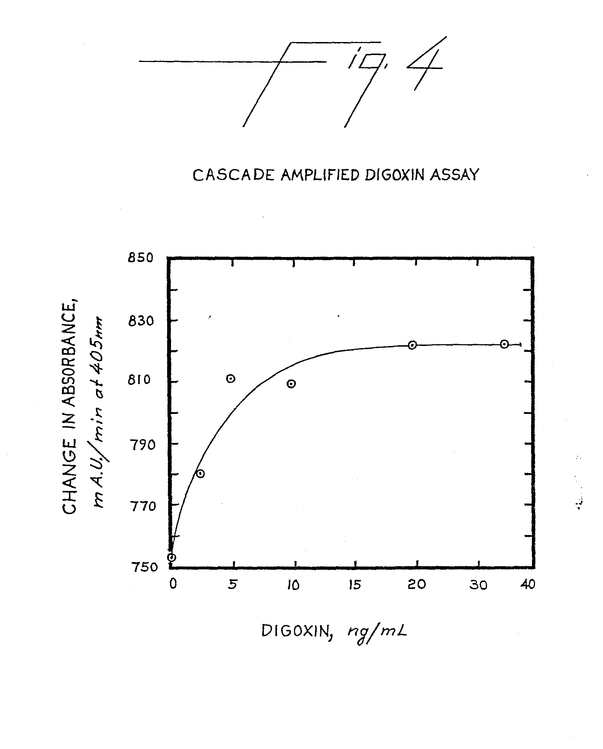

FIG. 1 is a standard assay curve for gentamicin analysis which relates the change in absorbance to gentamicin concentration in the test medium. FIG. 2 is a standard assay curve for theophylline analysis which relates the change in absorbance to theophylline concentration in the test medium. FIG. 3 is a trypsin assay curve, with and without chymotrypsinogen cascade amplification, which relates the change in absorbance to trypsin concentration in the test medium. FIG. 4 is a standard assay curve for digoxin which relates the change in absorbance to digoxin concentration in the test medium.

## DETAILED DESCRIPTION OF THE INVENTION

This invention provides a sensitive method and novel materials for detecting or determining the amount of a wide variety of organic substances present in clinical, environmental, and other test samples. In the context of this disclosure, the following terms shall be defined as follows: "analyte" is the substance, or group of substances, whose presence or amount thereof in liquid medium is to be determined, which additionally have the capability of being chemically attached to a zymogen substance to form a conjugate of an analyte and a zymogen; "analyte derivative" refers to both unmodified and chemically modified analyte molecules, and/or their chemical combinations with spacer arms, which can be chemically attached to zymogen molecules by covalent, ionic, or other bonding techniques; "spacer arm" is a bifunctional molecule used to chemically attach an analyte derivative to a zymogen molecule while maintaining some distance between the two molecular entities; "zymogen" is any substantially inactive protein precursor of an enzyme, polypeptide hormone, or structural biomolecule which is activated irreversibly by limited proteolysis; "enzymatic activator" is a substance, or group of substances, which reacts enzymatically with zymogen, conjugate of an analyte and a zymogen, or conjugate of a binding agent and a zymogen to yield active zymogen-derived-enzyme; "nonenzymatic activator" is a substance, or group of substances, which react nonenzymatically with zymogen, conjugate of an analyte and a zymogen, or conjugate of a binding agent and a zymogen to yield active zymogen-derived-enzyme; "conjugate of an analyte and a labeling substance" is any product in which analyte, analyte derivative, or analyte analog is attached, directly or indirectly, to a labeling substance including zymogen, enzymatic activator, or nonenzymatic activator; "zymogen-derived-enzyme" is any product derived from a conjugate of an analyte and a zymogen, a conjugate of a binding agent and a zymogen, or from a zymogen by limited proteolysis caused by an enzymatic activator, or by conformational change induced by a nonenzymatic activator, which can produce, or trigger the production of, detectable marker material; "binding agent" is any substance, or group of substances which has specific binding affinity for the analyte to the exclusion of other substances such that reaction occurs between a particular analyte and binding agent to form a bound complex; "conjugate of a binding agent and a labeling substance" is any product in which binding agent, or a derivative of binding agent, is attached directly or indirectly, to a labeling substance including zymogen, enzymatic activator, or nonenzymatic activator; "corresponding bioreactive substance" is the zymogen, enzymatic activator, or nonenzymatic activator which is appropriate for reaction with conjugate of an analyte or a binding agent and labeling substance to yield an active zymogen-derived-enzyme; "insolubilized binding agent" is binding agent chemically or physically attached to an insoluble, inert matrix; "reaction matrix" is a suspension of insolubilized binding agent and liquid medium confined in a layer; "insolubilized analyte" is analyte chemically or physically attached to an insoluble, inert matrix; "analyte analog" is any substance, or group of substances, which behaves essentially the same as the analyte with respect to binding specificity for the binding agent for analyte; "marker material" is an enzyme substrate which is not detectable by the instrumental system utilized until converted by zymogen-derived-enzyme, or final enzyme product of a reaction cascade, to a form detectable by the

13         **0123265**

instrumental method; and "cascade" is a sequence of coupled reactions in which the product of one reaction is the catalyst for the next.

The incorporation of an enzyme cascade initiated by a zymogen-derived-enzyme in a method for biochemical assay is highly significant. The outcome of a cascade is an amplification of the number of detectable product molecules since one molecule of the first product generated can catalyze the production of numerous molecules of the second, etc. In the case of an enzyme cascade, where the turnover numbers are large, great amplification may be achieved. This amplification results in major increases in sensitivity of the assay method.

The present method may be applied to the detection of any analyte for which a binding agent exists. The binding agent may consist of a polyclonal or monoclonal antibody in the form of whole antiserum or ascites fluid, an IgG fraction, or as affinity-purified monospecific material, or of other specific binding proteins like lectins and protein A. Usually it will be an IgG fraction, which in some cases may be further purified by immunoaffinity chromatography. In some cases, active antibody fragments, e.g., Fab or Fab', may be used. The quantitative measurement aspect of the invention results from the fact that free analyte present in the test sample and the conjugate of an analyte and a zymogen are both capable of reacting in a competitive fashion with binding agent. In a homogeneous immunoassay in the absence of free analyte, the concentration of the process components is adjusted such that there is minimal production of zymogen-derived-enzyme and hence minimal production of marker material. In the presence of free analyte, uncombined conjugate of an analyte and a zymogen exists which can be converted to active zymogen-derived-enzyme by activator, thereby resulting in the production of marker material at a rate and in an amount proportional to the amount of free analyte initially present. An amplification is obtained in the measurement process due to the fact that a single interaction between conjugate of an analyte and a zymogen and activator can result in the production of a large number of marker molecules, either directly or through the action of a coupled zymogen-to-enzyme cascade.

In a competitive heterogeneous immunoassay, conjugate of an analyte and a zymogen or an analyte and an activator competes with sample analyte for a limited number of insolubilized anti-analyte binding sites. The proportions of the process components are adjusted so that in the absence of sample analyte, essentially all of the labeled analyte is bound by the solid phase. Either the bound or the free fraction can be quantified. Where the labeling substance is a zymogen, activator and substrate reagent (which can be another zymogen and/or a chromogenic substrate) are added to produce a detectable marker material. Where the labeling substance is an activator, one or more zymogens and a chromogenic substrate reagent for the last zymogen in the cascade are added.

In a noncompetitive heterogeneous immunoassay, a conjugate of an antibody and a zymogen or of an antibody and an activator is employed. The method of assay can be as described in U.S. Patents 3,654,090 or Re. 31,006 or in U.S. Patent 4,434,236, all of which are hereby incorporated by reference. Quantification of either the bound or the free labeled species is accomplished by addition of the appropriate reagents as described above.

Various methods or protocols may be employed in assaying for a wide variety of analytes. The determination of the amount of analyte initially present in the test sample can be carried out by correlation with either the rate or extent of formation of detectable marker material. Incubation and contacting times of the reagents can vary from 1 to 60 minutes at temperatures in the range of from about 4°C to 45°C and a pH in the range of about 4 to 10. The measurement can be carried out manually, or with reagents packaged for use with an automatic clinical analyzer.

To illustrate the instant invention and a typical homogeneous assay procedure, an aliquot of unknown analyte is added to the buffer incubation medium. A known amount of antibody specific for the analyte is added to the medium and briefly incubated prior to the addition of a known amount of conjugate of an analyte and a zymogen. After further brief incubation, a known amount of activator is added. After further brief incubation, chromogenic or fluorogenic substrate for zymogen-derived-enzyme is added and the amount of substrate conversion by zymogen-derived-enzyme is monitored as a function of time. Comparison with a standard curve relating analyte concentration to substrate conversion obtained under the same conditions of time intervals and reagent amounts enables the determination of the unknown amount of analyte. The individual and frequently unique properties of the specific analyte and its associated binding agent can be utilized to optimize the conditions for the measurement.

Analyte

This invention can be applied to the detection and measurement of a broad variety of analytes for which binding agents are available. Analytes of particular interest include those drugs and hormones with either very low concentrations in biological fluids or with narrow therapeutic ranges. The cardiac steroid, digoxin, satisfies both criteria since levels below 0.8 ng/mL (nanograms per milliliter) in human serum are ineffective for treating cardiac arrhythmia while levels above 2.0 ng/mL are often toxic. Other analytes which fall into this category include vitamin $B_{12}$, folate, and most of the steroid, peptide, and protein hormones.

Analytes such as myoglobin normally have very low levels in serum that can rise dramatically after myocardial infarction and are therefore diagnostic of this condition. Analytes such as microbial and cancer cell markers would generally be low in concentration since early detection (prior to prolonged cell growth) is highly desirable. Aminoglycoside drugs, barbiturate drugs, and many of the miscellaneous drugs such as theophylline are relatively high in concentration with µg/mL (microgram/milliliter) levels but have narrow therapeutic ranges. Analytes of interest may be contained in various liquid media including, but not limited to, buffer solutions, serum, plasma, urine, blood, and cerebrospinal fluid. Table I lists a variety of analytes of particular interest in practicing the instant invention.

**0123265**

Table I

ANALYTES

| Alkaloid Drugs | Barbiturate Drugs (cont.) |
|---|---|

**Alkaloid Drugs**

benzoyl ecgonine
cocaine
codeine
dextromethorphan
heroin
lysergic acid
morphine
quinidine
quinine

**Aminoglycoside Drugs**

amikacin
gentamicin
kanamicin
neomicin
tobramicin

**Antibiotic Drugs**

actinomycetin
caromycin
chloramphenicol
chloromycetin
chlortetracycline
erythromycin
oxytetracycline
penicillin
polymyxin B
terramycin
tetracycline
streptomycin

**Barbiturate Drugs**

diphenylhydantoin
ethosuximide
phenobarbital

**Barbiturate Drugs (cont.)**

primidone
secobarbital

**Marijuana Derivatives**

cannabinol
tetrahydrocannabinol

**Metabolites**

galactose
phenylpyruvic acid
porphyrin
spermine

**Miscellaneous Drugs**

amitriptyline
anticholinergic drugs
antihistamines
atropine
butyrophenones
caffeine
carbamazepine
chloropromazine
epinephrine
griseofulvin
imipramine
L-dopa
lidocaine
meperidine
meprobamate
methadone
N-acetyl procainamide
narceine
nortriptyline
oxazepam
papaverine

Table I (continued)

| Miscellaneous Drugs (cont.) | Toxics in Food (cont.) |
|---|---|
| procainamide | ochratoxin |
| propanolol | patalin |
| prostaglandins | penicillic acid |
| tegretol | tricothecene toxin |
| theophylline | zearclonone |
| serotonin | |
| valproic acid | |

**Peptide Hormones**

**Vitamins**
biotin
folic acid
thiamine

adrenocorticotropin (ACTH)
angiotensin
met- and leu-enkephalin
oxytocin
thyroxine
triiodothyronine
vasopressin

vitamin A
vitamin $B_2$
vitamin $B_6$
vitamin $B_{12}$
vitamin C
vitamin D
vitamin E
vitamin K

**Pesticides**

carbamate pesticides
thiophosphate pesticides
polyhalogenated biphenyl
   pesticides
polyhalogenated sulfonamide
   pesticides

**Protein Hormones**

chorionic gonadotropin
chorionic thyrotropin
glucagon
insulin
nerve growth factor
parathyroid hormone
placental lactogens
prolactin
proinsulin
relaxin

**Steroids**

adrenocorticol steroids
androgens
bile acids
digoxin
digoxigenin
diethylstilbestrol
estrogen
gestrogen

**Proteins**

albumin
$\alpha_1$-acid glycoprotein
$\alpha_1$-glycoprotein
$\alpha_1$-lipoprotein
$\alpha_2$-glycoprotein
$\alpha_2$-lipoprotein
ß-lipoprotein

**Toxins in Food**

aflatoxins
ipomeamerone
mycotoxins

Table I (continued)

Proteins (continued)

ß-glycoprotein
C-reactive protein
fibrin split products
fibrinogen
immunoglobulin A
immunoglobulin D
immunoglobulin E
immunoglobulin G
immunoglobulin M
haptoglobin
hemoglobin
ceruloplasmin
cholinesterase
hemopexin
myoglobin
rheumatoid factor
thyroxine-binding globulin
transferrin
transcortin
plasminogen

specific antibodies
coagulation factors

Microbial Surface Markers

bacterial antigens
fungal antigens
parasitic antigens
viral antigens

Cancer Cell Markers

carcinoembryonic antigen
gangliosides
α-fetoprotein

Zymogens

A large number of zymogens exist which may be employed in the present invention. The utilization of the activation of a zymogen in homogeneous and heterogeneous assays provides many advantages. In the homogeneous mode, where the assay does not depend on the inhibition of existing enzymatic activity, background activity or "noise" can be minimized, thereby increasing the precision of the measurement. Zymogens are also highly stable in diverse chemical environments minimizing the need to recalibrate the standard assay curve in either homogeneous or heterogeneous immunoassays.

Zymogens are convertible irreversibly by limited proteolysis to a variety of bioactive molecules. While most known zymogens are activated to enzymes, there are many examples in which the zymogen is convertible to a hormone possessing systemic activity, or to a molecule which can undergo self assembly to produce structural cell material. A number of such zymogen/zymogen-derived-enzyme pairs are listed in Table II.

## Table II

| Zymogen | Bioactive Molecule |
|---|---|
| trypsinogen | trypsin |
| chymotrypsinogen | chymotrypsin |
| procolipase | colipase |
| prophospholipase | phospholipase |
| prorennin | rennin |
| procarboxypeptidase A | carboxypeptidase A |
| procarboxypeptidase B | carboxypeptidase B |
| kininogen | kinin |
| proelastase | elastase |
| prekallikrein | kallikrein |
| pepsinogen | pepsin |
| plasminogen | plasmin |
| fibrinogen | fibrin |
| prothrombin | thrombin |
| plasminogen proactivator | plasminogen activator |
| C1s | C1s (activated) |
| proacrosin | acrosin |
| coagulation factor XI | coagulation factor $XI_a$ |
| coagulation factor XII | coagulation factor $XII_a$ |
| coagulation factor XIII | coagulation factor $XIII_a$ |
| procollagenase | collagenase |
| prococoonase | cocoonase |
| angiotensinogen | angiotensin |
| proinsulin | insulin |
| proparathyroid hormone | parathyroid hormone |
| proglucagon | glucagon |
| procollagen (soluble) | collagen (insoluble) |
| prochitin synthetase (yeast) | chitin synthetase |
| viral coat protein precursors (bacteriophage) | assembly-competent viral protein |
| calcium/calmodulin dependent enzymes | calcium independent enzymes |
| $NAD^+$ kinase ($Ca^{++}$ dependent) | $NAD^+$ kinase ($Ca^{++}$ independent) |
| Ca++/Mg++ATPase, vertebrate muscle ($Ca^{++}$ dependent) | ATPase (Ca++ independent) |
| Cyclic nucleotide phospho-diesterase (Ca++ dependent) | Cyclic nucleotide phospho-diesterase (Ca++ independent) |

0123265

Conjugates of an Analyte and a Labeling Substance

The present invention utilizes novel conjugates of an analyte and a labeling substance for homogeneous and competitive heterogeneous immunoassays which can be synthesized by covalently linking labeling substances included in Table II with analytes listed in Table I. The conjugate of an analyte and a labeling substance can be synthesized by a variety of routes [see, for example, G. F. Means and R. E. Feeney, Chemical Modification of Proteins, 1971]. Conditions for covalently attaching an analyte, including its derivatives and analogs, to a zymogen depend upon the particular molecular architecture of both types of molecules. For the most part, zymogens are composed of amino acids which contain functional groups such as amino, carboxyl, hydroxyl, sulfhydryl, and others to which an analyte with its own functional groups could be directly or indirectly attached. The attachment chemistry can vary depending upon the functional groups involved, the number of analyte molecules to be attached per zymogen, and the desirability of including a "spacer arm" between the analyte and the zymogen. In addition to the above methods which label most or all of a particular amino acid residue, site-specific attachment methods are known for several zymogens [Radhakrishnan et al., Biochemistry, Vol. 8, 4020 (1969) and Magee et al., Biochem. J., Vol. 197, 239 (1981)]. For trypsinogen, the preferred sites of attachment include amino groups, especially the N-terminal amino group.

The chemical methods involved in attaching analyte molecules, or their derivatives or analogs, to the zymogen are known to those skilled in the art. The important features of a particular conjugate of an analyte and a zymogen are that the conjugate be stable under conditions of storage and use, i.e., that it not spontaneously release significant amounts of analyte or analyte derivative or analog, that it react with binding agent, and that it interact with activator to produce active zymogen-derived enzyme. These properties are generally obtained by attaching a minimal number of analyte, analyte derivative, or analyte analog molecules to zymogen. While covalent bonds have been found to insure these properties, bonding can involve other types of interactions which result in stable combinations of analyte and zymogen, such as ionic or hydrophobic bonds. Such bonding methods are within the scope of this invention. Additionally, the molecular structure of the analyte derivative actually attached to the zymogen to produce the conjugate can vary somewhat depending on the particular bonding reactions to be utilized and the possible use of a spacer arm. Such molecular structural variations to achieve attachment are not critical to characterize the conjugate of an analyte and a zymogen or to practice the invention.

The present invention can utilize a conjugate of an analyte and an activator in the heterogeneous mode. Such conjugates can be synthesized by a variety of routes known in the art. The analyte, or a derivative or analog thereof, is preferably attached covalently to the activator through such functional groups as amino, carboxyl, hydroxyls, sulfhydryls, etc. In general, one wishes to attach as many molecules of activator per analyte as possible, consistent with preserving the activity of each. Although covalent attachment is generally preferred for reasons of stability, it is possible that noncovalent attachment, as through hydrophobic or ionic bonds, can result in adequately stable conjugates.

## Conjugates of Binding Agent and Labeling Substance

The instant invention provides novel reagent means for the practice of noncompetitive heterogenous immunoassays in which a zymogen or an enzymatic or nonezymatic activator is coupled to a binding agent for a specific analyte. The binding agent (see below) is typically an antibody. The means of coupling is generally the same as described above for the synthesis of conjugates of analyte and a labeling substance.

For noncompetitive heterogeneous immunoassays, one generally wishes to couple as many molecules of the labeling substance to the binding agent as possible, consistent with preserving the specific activity of both components. This is in contrast to the labeling strategy employed in synthesizing conjugates of an analyte and a zymogen for use in homogeneous and competitive heterogeneous immunoassays, such as described above. In a conjugate for a competitive immunoassay, one wants to attach the minimum number of analyte molecules per zymogen in order to maximize sensitivity.

The free sulfhydryl groups present on Fab' fragments of immunoglobulins; the sulfonate groups present on sulfitolyzed half-molecules of immunoglobulins; and the sugar residues in the Fc region of immunoglobulins provide convenient sites at which the labeling substance can be attached without significantly affecting immunoreactivity. Amino, carboxyl, serine, and tyrosinyl groups distributed throughout the immunoglobulin molecule can also be utilized.

**0123265**

It is also desirable to utilize functional groups on the labeling substance which are not critical for its specific activity. For example, if the labeling substance is the specific enzymatic activator enteropeptidase, a nonessential sulfhydryl group can be exposed by limited reduction and used for coupling. If the labeling substance is the zymogen trypsinogen, the preferred sites of attachment include 15 of the 16 available amino groups, especially the N-terminus, as described above (the pendant amino group of the lysine residue closest to the N-terminus is part of the activation site).

While covalent attachment is generally preferred because of its presumably greater stability, there can be cases in which it is advantageous to utilize noncovalent or reversible covalent bonds. Disulfide bonding is a good example of covalent bonding which is reversible by reduction.

Where the labeling substance is an enzymatic activator having a broad spectrum of proteolytic activity, e.g., trypsin, it may be desirable to perform the coupling at low pH or in the presence of removable protease inhibitors in order to prevent the label from attacking the antibody.

Binding Agents

Analytes (as well as conjugate of an analyte and a zymogen) and their specific binding agents rapidly interact and combine to form a tightly bound complex. Antisera for the analytes shown above are known. To produce an immunogen, the smaller analytes (molecular weight less than 5000 daltons) are generally haptenated (covalently conjugated in immunologically active form), to proteins like human or bovine serum albumin, or to keyhole limpet hemocyanin. The antibody can be used in this assay as a serum, a partially purified immunoglobulin fraction, as an immunopurified monospecific antibody fraction, or as monoclonal antibody. Monovalent fragments of antibody may also be used. The preparation of these various antibody fractions are well known in the literature. [Weir, D.M., Handbook of Experimental Immunology, Blackwell Science Publication, Oxford 1978, pp. 6.1 to 10.5.]

For some analytes, specific binding agents can be substituted for specific antibodies in these assays. For example, physiological hormone receptors extracted from tissue homogenates can be used in place of antibodies to detect and quantify hormones. For glycoproteins, specific plant or animal lectins can be used. Similar binding agents also exist for many vitamins (e.g., biotin/avidin and vitamin $B_{12}$/intrinsic factor) which exhibit very high affinity. Such nonantibody binding agents are advantageous in that they are naturally occurring, sometimes monovalent, and have high affinity and specificity.

Activator

Activator for conversion of zymogen, or zymogen conjugate, to zymogen-derived-enzyme is usually a protein and frequently an enzyme. Enzymatic activators would include enteropeptidase, whose sole biological activity is to convert trypsinogen to enzymatically active trypsin, or trypsin which converts chymotrypsinogen to chymotrypsin. Streptokinase is an example of a nonenzymatic activator which can be utilized for activation of plasminogen to plasmin. Activator preparations may contain contaminating proteases or inhibitors and may require substantial purification before use in the method of this invention.

Purification can be achieved in several ways, including negative affinity chromatography, gel filtration, ion exchange chromatography or a combination of the techniques. The particular technique utilized will depend on the nature of the activator and of the impurities to be removed, as well as the degree of purity desired.

Zymogen Activation Cascade and Substrate Reagent

A number of biological cascades, e.g. the coagulation, complement and digestive cascades, are known which could be utilized or adapted for the present invention. The purpose of integrating a zymogen activation cascade with a diagnostic assay is to achieve enhanced measurement sensitivity. In general, substrate reagent is convertible by the final enzyme product of the reaction cascade to a marker material which is detectable. To function in the above manner, the selected zymogen activation cascade need only be triggered by the zymogen-derived-enzyme produced by activation of the conjugate of an analyte and a zymogen. Such cascades employing excess ultrapurified unlabeled zymogen can also be used to increase sensitivity in conjunction with analyte-labeled enzymes such as that disclosed in U.S. Patent 3,817,837 which is herein incorporated by reference.

Homogeneous Assay Method using Conjugate of an Analyte and a Zymogen

The assay of this invention can be performed manually or it can be adapted to a variety of automated or semi-automated instrumentation, for example, the aca$^{TM}$ discrete clinical analyzer (available from E. I. du Pont de Nemours and Company).

The homogeneous assay method using a conjugate of an analyte and a zymogen is typically performed by mixing conjugate of an analyte and a zymogen, analyte-specific antibody, sample suspected of containing analyte, and activator sequentially or simultaneously in a suitably buffered medium (pH 4-10, more often 6-9). After a suitable incubation period, usually 1 to 5 minutes at a temperature between 0° and 45°C, preferably between 18° and 38°C, a signal-producing substrate reagent (usually a chromogenic substrate) for the zymogen-derived enzyme is added. After another incubation period, usually from 5 seconds to 30 minutes and most often from 10 seconds to 5 minutes, the rate or extent of signal formation is measured visually or spectrophotometrically. In some cases, activator is added prior to or simultaneously with substrate reagent after the initial incubation period.

In the cascade mode, the assay is performed analogously except that the second zymogen is added along with the conjugate, antibody, analyte, and activator.

If the assay is carried out in an aca$^{TM}$ analytical test pack (described in Re. 29,725, issued August 8, 1978 to Johnson et al., which is hereby incorporated by reference), sample suspected of containing analyte is added to the test pack in the filling station of the instrument, followed by injection of a volume of buffer sufficient to bring the initial pack volume to 5 mL. Sample size can vary between 20 µL and 500 µL. The buffer can be any aqueous buffer between pH 4 and 10, typically between pH 6 and 9, and can contain salts, surfactants, organic solvents, preservatives, signal generating reagents, etc. Analyte-zymogen conjugate, analyte-specific antibody, and activator (and second zymogen, if the assay performed is a cascade) are added automatically, usually in breaker/mixer I. Each reagent can be released from a separate dimple either as a liquid or a tablet; alternatively, certain reagents can be mixed in the same dimple as, for example activator and analyte-specific antibody. After a 222 to 232 second incubation at 37°C, the pack enters breaker/mixer II where additional reagents can be added. Typically, a chromogenic substrate for the zymogen-derived enzyme is added; other signal generating reagents as, for example, DTNB or turbidity generating substrate can also be added at this time. In some applications, activator can be added along with signal generating substrate at breaker/mixer II, instead of at breaker/mixer I. After a 29 to 39 second incubation, the pack enters the photometer where signal formation (measured as a rate or endpoint) is determined 17 seconds later at the desired wavelength(s).

For extremely low concentrations of analyte, it can be desirable to preincubate certain reactants outside the pack, e.g., sample and analyte-specific antibody. Depending upon the origin of the liquid medium containing the analyte of interest, the sample can contain interferents which can adversely interact with the reagents utilized in the assay, e.g., protease inhibitors in serum. In such cases, the sensitivity of the assay can be enhanced by performing pretreatments such as acidification, heating, ion exchange chromatography, precipitation, exposure to antibody directed against the interferent, deproteinization, or detergent denaturation of the test sample. One can also add such substances as a decoy protease, e.g. chymotrypsin or elastase, to inactivate the interferent. These substances can also be added to the test pack either in the form of additional reagents contained in the dimples, or in the pack header.

In another embodiment, zymogen activation reaction or activation cascade can be coupled to the homogeneous assay of U.S. Patent 3,81,937, which employs an analyte-enzyme conjugate. If the enzyme is chosen so as to be capable of activating a zymogen, binding of anti-analyte to the analyte-enzyme conjugate will modulate the ability of the conjugate to activate excess unlabeled zymogen. In general, anti-analyte can be expected to inhibit activation, but it is conceivable that under certain circumstances activation might be enhanced. Use of zymogen activation in such an immunoassay can be expected to yield a substantial increase in assay sensitivity, while coupling an activation cascade should yield an even greater increase in sensitivity.

Heterogeneous Assay Method

The assay of this invention can also be performed in a heterogeneous mode.

Where the assay is performed in a competitive heterogeneous mode, the conjugate consists of an analyte and a labeling substance chosen from the group consisting of a zymogen, an enzymatic activator which is capable of activating a zymogen, and a nonenzymatic activator which is capable of activating a zymogen. A limiting amount of conjugate is mixed with a sample suspected of containing analyte and the mixture is allowed to react with a limiting amount of immobilized anti-analyte and either the free or the bound reaction can be measured. Where the labeling substance is a zymogen, an activator and at least one substrate for zymogen-derived-enzyme will be required to generate detectable marker material. Where the labeling substance is an activator, at least one zymogen and a chromogenic substrate for zymogen-derived-enzyme will be required to generate detectable marker material.

Where the assay is performed in a noncompetitive mode, the conjugate consists of a specific binding partner for the analyte and either a zymogen or an activator.

In the mode disclosed by U.S. Patent 3,654,090, which is hereby incorporated by reference, sample suspected of containing analyte is mixed with labeled anti-analyte, present in molar excess over analyte. The resultant mixture is passed over excess insolubilized analyte to which any unreacted antibody from the first step will bind. Labeled antibody/analyte complex can flow freely through the matrix and can be quantified as above by addition of the appropriate reagents.

In the mode disclosed by U.S. Patent 4,434,236, the assay is performed by passing sample suspected of containing analyte over insolubilized analyte-analogue having labeled antibody immunochemically bound thereto. Sample analyte quantitatively displaces labeled antibody from the matrix and the amount of label so displaced is quantified as above by the addition of the appropriate reagents.

In the mode disclosed by U.S. Re. 31,006, which is hereby incorporated by reference, sample suspected of containing analyte is allowed to react with excess insolubilized anti-analyte. Analyte bound to the solid pase is then reacted with labeled anti-analyte to form a sandwich and the bound label is quantified.

## EXAMPLES

Unless specified otherwise, in all statements of assay conditions and preparation of reagents, temperature is expressed in °C and concentrations referred to as percentages are by weight/volume. The symbol mA.U. is defined to be milliabsorbance units used to quantify changes in absorbance.

## EXAMPLE I

### TRYPSINOGEN-ACTIVATION IMMUNOASSAY FOR GENTAMICIN

#### A.  Synthesis of Gentamicin-trypsinogen Conjugate

To a 15 mL tube in an ice-water bath was added 9.4 mL of 0.1M MES buffer (2[N-morpholino]ethanesulfonic acid), pH 6.5 containing 10 mg/mL gentamicin sulfate. One hundred μL of trypsinogen (12.5 mg/mL in ice-cold 5 mM formate, pH 3.0) was added with gentle mixing, followed by the dropwise addition of 500 μL of 1% glutaraldehyde (in 0.1 M MES, pH 6.5) with rapid mixing. The solution was then rocked for 30 minutes at room temperature.

The faintly yellow, clear solution (pH 6.3) was transferred to a 20 mL beaker with magnetic stirrer and 1.2 mL of sodium borohydride (1 M in distilled water, freshly prepared) was slowly added with gentle stirring. After 30 minutes of stirring, the alkaline solution (pH 9.0 - 9.2) was slowly titrated with 1 M formic acid until the pH was 6.0 ± 0.1. The solution was then dialyzed at 4°C against 3 2-liter changes of 5 mM formic acid (8-18 hours for each dialysis).

The dialyzed conjugate was assayed for activation by enteropeptidase in the aca$^{TM}$ discrete clinical analyzer. A 50 μL sample of diluent (50 mM Tris HCl, 0.1% w/v $NaN_3$, pH 8.3, at 25°) was automatically injected into an analytical test pack in the filling station of the instrument, followed by 4.95 ML of diluent containing 1 mg/mL 5,5'-dithiobis 2-nitrobenzoic acid (DTNB). 250 µg crude enteropeptidase (from Sigma in 10 mM MES, 0.1% w/v $NaN_3$, pH 6.5) was added in breaker/mixer I. In this assay, enteropeptidase obtained from Sigma did not require purification. Each test pack also contained 1.4 mg of thiobenzyl-benzyloxycarbonyl-L-lysinate (ZLTBE, product of Penninsula Laboratories) which was added in breaker/mixer II. Duplicate packs were prepared which contained in addition 5 µg native trypsinogen or conjugate in 50 µL of 5 mM formic acid, which was added at breaker mixer I. Both enteropeptidase and trypsin-like enzymes, but not trypsinogen, rapidly cleave ZLTBE to yield (with DTNB) a colored product which absorbs at 405 nm. The amount of trypsinogen activation can be determined from the difference in the rate of cleavage of ZLTBE in the presence and absence of trypsinogen. With the above level of enteropeptidase, the conjugate retained 48% of its activatability in the aca[TM] discrete clinical analyzer method compared to unlabeled trypsinogen.

To ascertain whether the conjugate was inhibitable by anti-gentamicin, packs were prepared as described above but with 2.45 mg (50 µL) of an IgG fraction of antigentamicin (Atlantic Antibodies) added in breaker/mixer I. The conjugate was shown to be at least 50% inhibitable by antibody. Unlabeled trypsinogen was not significantly inhibited by antibody.

### B. Immunoassay for Gentamicin

A standard curve for gentamicin was generated using gentamicin sulfate standards prepared in diluent (lacking DTNB) to contain 0, 300, and 1500 µg/mL

gentamicin. 50 μL of standard was added to the pack from the sample cup, followed by 4.95 mL of diluent containing 1 mg/mL DTNB. Analytical test packs were prepared exactly as described above with 5 μg conjugate except that 270 μg (5.5 μL) of IgG antibody was used. FIG. 1 shows the standard curve from this experiment in $\frac{mA.U.}{min}$ at 405 nm versus gentamicin concentration in the sample.

## EXAMPLE II

## TRYPSINOGEN-ACTIVATION IMMUNOASSAY FOR THEOPHYLLINE

### A. Antibody Purification

IgG antibodies to theophylline were purified from rabbit serum by a combination of ammonium sulfate precipitation and ion exchange chromatography.

Four and one-half parts of saturated (22°C) ammonium sulfate were slowly added with gentle stirring to five and one-half parts of rabbit antiserum (Miles Laboratories) at room temperature. After 15 minutes, the precipitated immunoglobulins were pelleted by centrifugation at 10,000g for 10 minutes, then redissolved to one-tenth the original serum volume in distilled water. This immunoglobulin (Ig) solution was dialyzed overnight at 4°C against 100 volumes of 5 mM Tris HCl, 50 mM NaCl, 0.1% NaN$_3$, pH 7.6.

After dialysis, the Ig solution (7.5 mL) from 75 mL of antiserum was applied to a 50 mL column (2.5 cm i.d.) of DEAE-Sephacel (Pharmacia; beaded cellulose derivatized with diethyl aminoethyl groups) equilibrated in the buffer used for dialysis above. Fractions (3 mL) were collected during elution with the same buffer and the absorbance at 280 nm was determined. The peak fractions were pooled and precipitated with an equal volume of saturated ammonium sulfate at room temperature. The resultant precipitate was collected by centrifugation, redissolved, and dialyzed as described above. IgG recovery was 258 mg as calculated using an extinction coefficient ($E_{1cm}^{1\%}$ 280) of 14.

B. Assay and Purification of Enteropeptidase (E.C. 3.4.21.9)

Assay for Enteropeptidase Esterase Activity & Purity

Enteropeptidase from porcine intestine obtained from commercial sources exhibited variable amounts of trypsin-like contaminants which can interfere in trypsinogen activation immunoassays. To determine the purity and activity of enteropeptidase, a rapid, sensitive chromogenic assay was developed which eliminates interference from trypsin-like contaminants.

Enteropeptidase (obtained from Miles Laboratories and from Sigma) was assayed with the aca$^{TM}$ discrete clinical analyzer. A 500 µL sample of enteropeptidase containing 0-5 mg protein/mL in diluent (50 mM Tris HCl, 5 mM $CaCl_2$, 0.1% w/v $NaN_3$, pH 8.3 at 25°C) was automatically injected into an analytical test pack in the filling station of the instrument, followed by 4.50 mL of diluent containing 1 mg/mL 5,5'-dithiobis (2-nitrobenzoic acid) (DTNB). Each test pack contained 1.4 mg of thiobenzyl-benzyloxycarbonyl-L-lysinate (ZLTBE, product of Penninsula Laboratories) which was added in breaker/mixer II. Duplicate packs were prepared which contained in addition to ZLTBE 500 µg of soybean trypsin inhibitor (SBTI, available from Sigma) in 50 µL diluent, which was added at breaker/mixer I. Both enteropeptidase and trypsin-like enzymes cleave ZLTBE to yield, in the presence of DTNB, a colored product which absorbs at

0123265

405 nm. Trypsin-like enzymes, however, are rapidly inhibited by SBTI while enteropeptidase is not. The amount of contaminating trypsin-like enzymes in an enteropeptidase preparation can thus be determined from the difference in the rate of cleavage of ZLTBE in the presence and absence of a large excess of SBTI.

Table III compares the rate of cleavage of ZLTBE in the presence and absence of SBTI for unpurified enteropeptidase. One aca[TM] analyzer unit of enteropeptidase activity was defined as that amount of enzyme [approximately 1 nM in pack estimated from H. S. Savithri and A. Light, Biochem. Biophys. Res. Comm., 94: 360-363 (1980)] which yields 200 mA.U./min with ZLTBE and SBTI under the above described conditions.

### Table III
#### ENTEROPEPTIDASE ASSAY FOR PURITY

| Enteropeptidase* | | aca[TM] analyzer rate ( mA.U./min) | |
|---|---|---|---|
| Source | Protein conc., mg/ml | without SBTI | with SBTI |
| MILES LABS | 0 | 18 | 18 |
| | 0.125 | 1620 | 55 |
| | 0.250 | off-scale | 115 |
| | 1.0 | off-scale | 390 |
| | 5.0 | off-scale | 1835 |
| SIGMA | 0 | 18 | 18 |
| | 0.125 | 25 | 22 |
| | 0.250 | 40 | 37 |
| | 1.0 | 108 | 99 |
| | 5.0 | 480 | 470 |

* 500 µL sample for aca[TM] analyzer

It is clear from Table III that commercially available enteropeptidase can be contaminated by trypsin-like enzymes. While Sigma enteropeptidase contained very little trypsin-like contamination, trypsin inhibitory activity was present which interfered with trypsinogen activation immunoassays at levels above 250 μg enteropeptidase protein per test pack.

## Purification of Enteropeptidase by Ion Exchange Chromatography

170 mL of DEAE-Sephacel was equilibrated with 5 mM MES, 50 mM NaCl, 0.005% w/v thimerosal, pH 6.0 (column buffer) at 4°C in a 2.5 cm (i.d.) column. Crude enteropeptidase from Miles Laboratories (2 gm protein in 75 mL of column buffer) was dialyzed overnight at 4°C against 2 L of column buffer. The dialyzed enzyme solution was then applied to the column and eluted with a linear gradient of 50-500 mM NaCl in 5 mM MES, 0.005% w/v thimerosal, pH 6.0. 500 mL of 50 mM NaCl buffer was used. 6 mL fractions were collected and assayed for ability to activate trypsinogen.

The aca$^{TM}$ packs contained 1.4 mg ZLTBE released at breaker/mixer II plus 1.25 μg trypsinogen (in 50 μL 5 mM formate, 1% PEG 6000), released at breaker/mixer I. A 50 μL enteropeptidase sample was followed by 4.95 mL of diluent. Packs lacking trypsinogen and packs lacking trypinsogen but containing 500 μg SBTI were also run with the same samples. Trypsin-like contaminants eluted from the column immediately while enteropeptidase eluted at approximately 0.19 M NaCl. The peak fraction of purified enteropeptidase yielded 188 mA.U./min with ZLTBE alone, 155 mA.U./min with SBTI, and 1766 mA.U./min with trypsinogen.

Since 1.25 μg (10 nM) pure trypsin (Sigma) produced 2000 mA/min with ZLTBE, the trypsinogen-specific signal of 1578 mA.U./min represented 79% activation using 0.78 aca$^{TM}$

analyzer units of purified enteropeptidase.
Unpurified Sigma enteropeptidase (250 µg protein)
yielding 72 mA.U./min with ZLTBE and 68 mA.U./min
with SBTI (0.34 aca$^{TM}$ units) produced only 479
mA.U./min with trypsinogen (20% activation).

The peak fractions were pooled, quick-frozen
and lyophilized. The lyophilizate was redissolved in
one-tenth the original volume of distilled water and
dialyzed against 100 volumes of column buffer. After
dialysis, the purified enteropeptidase was stored
either at 4°C or quick-frozen and stored at -20°C.

C. Synthesis of Theophylline-trypsinogen Conjugate

Preparation of 8-(3-carboxypropyl)-1,3-
dimethylxanthine

The 8-substituted theophylline derivative,
8-(3-carboxypropyl)-1,3-dimethylxanthine, was
synthesized as follows: glutaric anhydride (6.8 g)
and 4,5-diamino-1,3-dimethyl pyrimidine-2,6-dione
(6.2 g) were refluxed under nitrogen in 15 mL of
N,N-dimethylaniline for 3 hours using a Dean-Stark
trap. The mixture was cooled, filtered, and the
solid product (2.6 g) was recrystallized twice from
water. The purified derivative had a melting point
of 254-255°C and the expected parent ion peak in the
mass spectrograph of m/e=266.

Conjugation to Trypsinogen

The 8-substituted theophylline derivative
(27 mg) was dispersed in 2.5 mL of absolute ethanol.
2.5 mL of distilled water was added, followed by 1 mL
of dimethyl sulfoxide (DMSO). To 1 mL of this
solution was added 100 µL of 1-ethyl-3-(3-dimethyl-
aminopropyl) carbodiimide (EDCI) which had been
dissolved at 100 mg/mL in distilled water just before
use. This solution was stirred at room temperature
for 30 minutes, then 1.1 mL of trypsinogen (15 mg/mL protein in 5 mM HCl, 0.2 mM benzamidine, pH 2.4) was added. The pH of the resultant solution was adjusted to 4.2 with 0.1 M NaOH. After 1 hour of gentle stirring, the reaction mixture was diluted 1:10 with 50 mM formic acid and transferred to dialysis tubing for dialysis against 2 L of 5 mM formic acid.

The coupling ratio, assuming a molar extinction coefficient for theophylline of 12,200 was estimated by determining the optical density at 280 nm for dialyzed conjugate, the equilibrated dialyzate, and a dialyzed trypsinogen control which lacked EDCI. From this calculation, an average of 1.1 moles of theophylline were estimated to be bound per mole of trypsinogen.

The ability of the conjugate to be activated by enteropeptidase was determined exactly as described for gentamicin-trypsinogen in Example I, except that 25 μg trypsinogen conjugate was used and diluent containing 5 mM $CaCl_2$. Activatability in the aca$^{TM}$ method was 26% compared to native trypsinogen using the indicated level of activator.

Purification of the Conjugate

The theophylline-trypsinogen conjugate was purified by ion-exchange chromatography. Carboxymethyl-cellulose (5 mL) was equilibrated with 10 mM citric acid, pH 3.7. The conjugate (10 mL) was applied to the column and eluted with a linear gradient of 10-250 mM citrate, pH 3.7 (100 mL total volume). A single major protein peak was eluted (maximum $A_{280}$=0.65) of which the early fractions contained the conjugate and the late fractions, small amounts of contaminating trypsin. Free theophylline eluted ahead of the peak. The early peak fractions were pooled and dialyzed against 5 mM formic acid.

Antibody inhibition of conjugate activation was assayed as described in Example IB, except that 20 nM (2.5 µg) of conjugate was used and enteropeptidase (250 µg protein, Sigma) was premixed in the reagent dimple with 625 µg of anti-theophylline IgG.  Two mg of human serum albumin was also released at breaker/mixer I.  Maximum inhibition by antibody was 30%.

### D.   Immunoassay for Theophylline

Theophylline (0-30 µg/mL) standards were prepared in a 0.9% saline, 0.1% azide solution containing 6% bovine serum albumin and 1% bovine gammaglobulin.  A 20 µL sample was injected automatically into analytical test packs, followed by 4.98 mL of diluent containing DTNB.  Each pack contained 2.5 µg theophylline trypsinogen conjugate, 2 aca$^{TM}$ analyzer units of enteropeptidase (purified as in Example IIB), and 38 µg of anti-theophylline IgG (purified as in Example IIA), all added in breaker/mixer I.  ZLTBE (1.4 mg) was added in breaker/mixer II.  The packs were processed on the aca$^{TM}$ analyzer according to standard operating parameters for enzyme rate measurements.  The standard curve obtained is shown in FIG. 2.

### EXAMPLE III

### IMMUNOASSAY FOR DIGOXIN

### A.   Preparation of Digoxin-Trypsinogen Conjugate

Synthesis of Oxidized Digoxin

Digoxin (8 mL, 16 mg/mL in absolute ethanol) and sodium periodate solution (8 mL, 16 mg/mL in distilled water, freshly prepared) were mixed in a screw-top tube and rocked for 1 hour at room temperature.  The solution cleared gradually.  To remove the periodate anion this solution was passed over a 10 mL column of AG-IX8 ion exchange resin (styrene divinylbenzene copolymer with quaternary ammonium groups) equilibrated in 50% (v/v) ethanol and then drained. An aliquot of the eluate was diluted 1:1000 in 50% (v/v) ethanol and the absorbance at 220 nm determined ($A_{220}$=0.18). Assuming a molar extinction coefficient of 18,000, the concentration of digoxin was approximately 10 mM (7.8 mg/mL). The column eluate was bubbled for one minute with dry nitrogen and stored at -20°C.

The extent of oxidation of digoxin was determined by spotting on GHL silica plates (Analtech; 250 μm thick, glass-backed plates coated with silica gel G) and developing the plates in chloroform:methanol:formic acid (70:20:2) or chloroform:methanol:ammonia (70:20:2). The plates were then sprayed with 5% (v/v) sulfuric acid in absolute ethanol and charred. Using chloroform:methanol:formic acid as the solvent, oxidized digoxin ran as a homogeneous band with $R_F$=.85. Using chloroform:methanol:ammonia, an additional minor spot was seen with a mobility corresponding to native digoxin ($R_F$=.75). The presence of aldehyde groups in the oxidized digoxin was confirmed by spraying the plates with Schiff's reagent (0.18% fuchsin, 0.56% sodium bisulfite).

Synthesis of the Conjugate

The following reagents were mixed gently in a screw top tube in the order indicated: trypsinogen (20 mL, 10 mg/mL in cold 5 mM formic acid), citric acid (1.6 mL of 1 M, pH 3,4,5 or 6), benzamidine (80 μL, 10 mM), distilled water (240 μL), and oxidized digoxin (4 mL, 10 mM). To each of these solutions was added 80 μL of 1 M sodium cyanoborohydride ($NaH_3BCN$) in water. The tubes were sealed and rocked for one hour at room temperature. A white precipitate formed which was removed by centrifugation. The supernatants were transferred to dialysis tubing and dialyzed at room temperature against 2 L of 50 mM formic acid for 4 hours. The dialysis solution was replaced with 5 mM formic acid and dialysis was continued for 18 hours at 4°C. A dense white precipitate formed in the bags and was removed by centrifugation. The supernatants containing the conjugates were stored at -20°C.

Chromogenic Assay to Determine the Ratio of
Digoxin to Trypsinogen in the Conjugate

A 1 mg/mL standard of oxidized digoxin in 50% (v/v) ethanol was prepared. A standard curve was generated by running duplicate tubes containing 0-50 μL of the above standard, sufficient 50% ethanol to achieve a 100 μL volume, and 2 mL of 49% (v/v) $H_2SO_4$. The tubes were stirred using a vortex mixer and read at 360 nm after a one hour incubation at room temperature.

From this standard curve, the concentration of digoxin in each of the dialyzed conjugates and in each of the last dialysis solutions was determined. There were an average of 5-7 digoxin molecules bound per trypsinogen molecule.

The ability of the dialyzed conjugate to be activated by enteropeptidase was determined as described in Example II, using 1.25 μg conjugate (in 50 μL 5 mM formic acid, 1% PEG 6000). Activation decreased with increased coupling pH, ranging from 37% at pH 3 to 24% at pH 6. DEAE-purified enteropeptidase yielded greater activation (up to 100% using 2 aca[TM] units and 0.25 μg conjugate).

Antibody inhibition was assayed as in Example II except that the conjugate was present in the sample cup at 25 μg/mL in 5 mM formic acid, 1% (w/v) PEG 6000. A 50 µL sample was injected into the pack, followed by 4.95 mL of diluent containing 1 mg/mL DTNB. Antibody (488 µg IgG, goat anti-digoxin-BSA obtained from Atlantic Antibodies) in 12.5 µL was diluted to 50 µL with human serum albumin (40 mg/mL in water) and added in breaker/mixer I.

Antibody inhibition was dependent on the pH at which the labeling was done. Maximum inhibition was 71% (pH 5) and minimum was 50% (pH 3).

Purification of the Conjugate

The conjugate was purified exactly as described in Example 5C, using a 4.0 mL column sample of dialyzed conjugate (typical maximum absorbance of peak fractions at 280 nm=0.7). Analysis of digoxin:trypsinogen coupling ratio as described above indicated 2-3 digoxins per trypsinogen in peak fractions.

Antibody inhibition assayed as described above was greater for leading peak fractions than for trailing peak fractions. Maximal inhibition was increased compared to unpurified conjugate. For example, using only 123 µg of IgG, maximal inhibition of the pH 6 conjugate increased from 67% to 74% after purification.

B. Chymotrypsinogen Ultra Purification

Crystallized chymotrypsinogen (650 mg) (from Worthington) was dissolved in 13 mL of ice cold 5 mM formic acid (pH 3.1). To this solution was added an equal volume of human $\alpha$-1-antiproteinase($\alpha$-1-antitrypsin from Sigma, chromatographically purified, 10.4 mg in 25 mM MES buffer, pH 6.0 at 4°C.)

After rapidly mixing the two solutions, incubation was carried out for 60 minutes at room temperature. The reaction was terminated by titrating to pH 3.0 with 1 M formic acid.

Ultrapurified chymotrypsinogen was assayed in the aca$^{TM}$ analyzer for residual chymotrypsin and for activation by dilute trypsin. aca$^{TM}$ test packs released at breaker/mixer I 50 µL of purified chymotrypsinogen solution (1.25 mg), plus 50 µL of 1 mg/mL human serum albumin in 5 mM formic acid containing a dilution series of 0-1.5 µg/mL trypsin (0-0.6 nM in the final reaction). The chymotrypsin chromogenic substrate, S-2586 (Kabi; 3-carbomethoxypropionyl-L-arginyl-L-prolyl-L-tryosine-p-nitroanilide hydrochloride) (0.7 mg in 50 µL 0.1% w/v NaN$_3$) was released at breaker/mixer II.

Packs were processed without samples, using 5.0 mL of diluent (50 mM Tris, 5 mM CaCl$_2$, 0.1% NaN$_3$, pH 8.55 at 37°) and standard instrument procedures for enzyme rate determination at 405 nm. Results are shown in FIG. 3.

Treatment of chymotrypsinogen with α-1-antitrypsin significantly decreased endogeneous chymotrypsin activity. Excess α-1-antiproteinase was denatured by the final low pH, and trypsin detection was improved greater than 500%, compared to direct detection with a comparable specific trypsin substrate (S-2444 from Kabi; L-pyroglutamyl-glycyl-L-arginine-p-nitroanilide HCl).

C. Enteropeptidase Purification with
Soybean Trypsin Inhibitor

Enteropeptidase (Miles Laboratories) was purified by negative affinity chromatography on an SBTI column to rapidly remove contaminating trypsin-like enzymes. 5 mL of SBTI-agarose (Miles Laboratories) was equilibrated in a 0.5 cm diameter column with cold 50 mM Tris HCl, 0.1% NaN$_3$, pH 8.1 (column buffer). Twenty-five mg of crude enteropeptidase (5 mg protein/mL in cold column buffer) was loaded and eluted with the same buffer. Two mL fractions were collected. The ability of purified enteropeptidase to activate trypsinogen was assayed in the aca$^{TM}$ analyzer using test packs which contained 13.25 µg trypsinogen (from Worthington) in 5 mM formic acid, added in breaker/mixer I, and 1.4 mg of ZLTBE, added in breaker/mixer II. A 50 µL enteropeptidase sample and 4.95 mL of diluent (50 mM Tris, 1 mg/mL DTNB, 0.1% NaN$_3$, pH 7.9 at 37°) were used.

Essentially complete removal of contaminating trypsin-like enzymes was achieved by this purification method. Maximum ZLTBE rate was 47 mA.U./min., while packs containing trypsinogen yielded >2300 mA.U./min. This purified enteropetidase still contained sufficient trace protease activity such that 2.0 aca$^{TM}$ analyzer units caused significant activation of chymotrypsinogen when added to packs prepared as described in B above but lacking trypsin. This chymotrypsinogen activation was largely eliminated by adding 2.5 µg SBTI to enteropeptidase in the reagent dimple.

#### D. Cascade-amplified Zymogen Activation Immunoassay for Digoxin

Digoxin standards (0-40 ng/mL) were prepared in 5 mM formic acid containing 1 mg/mL human serum albumin (pH 3.1), using digoxin dissolved as a stock solution (300 µg/mL) in ethanol. Packs were prepared as in B above using 50 µL of chymotrypsinogen except that a 500 µL digoxin standard was injected at the filling station followed by 4.5 mL of diluent. Instead of trypsin, 0.25 µg of trypsinogen-digoxin conjugate was released at breaker/mixer I. In addition, 2.0 aca$^{TM}$ analyzer units of enteropeptidase plus 2.5 µg SBTI were premixed in the reagent dimple with 10 µg goat anti-digoxin IgG (from Cappel Laboratories). This mixture was released at breaker/mixer I. FIG. 4 is a standard assay curve relating digoxin concentration to rate of change in absorbance.

> E. Immunoassay for Digoxin in Serum Using Chymotrypsin to Eliminate Protease Inhibitors

Ten normal human sera were assayed after adding digoxin (300 µg/mL stock solution in ethanol) to a final concentration of 5 or 10 µg/mL. Five normal sera were assayed without adding digoxin. A 20 µL sample was injected automatically into analytical test packs, followed by 4.98 mL of diluent.

Each pack contained 2.5 µg digoxin-trypsinogen conjugate (in 50 µL of 5 mM formate, 1 mg/mL HSA), 318 µg anti-digoxin IgG (described in D), 5 mg chymotrypsin (Sigma Type II, in 50 mL of 5 mM formate), all released at breaker/mixer I.

Released at breaker/mixer II were 2 aca$^{TM}$ analyzer units of enteropeptidase purified as described in C (but using lima bean trypsin inhibitor agarose from the United States Biochemical Company; 2 aca$^{TM}$ analyzer units in 50 µL of 5 mM Tris HCl, 50 mM NaCl, 0.1% azide, pH 7.5) and the chromogenic trypsin substrate S-2444 (from Kabi, L-pyroglutamyl-glycyl-L-arginine-p-nitroanilide HCl, 0.5 mg in 100 µL of 0.1% azide). Responses are shown in Table IV. Variation between sera is similar to variation between replicates of a single sample.

0123265

## Table IV

### IMMUNOASSAY FOR DIGOXIN
### IN SERUM USING CHYMOTRYPSIN TO
### ELIMINATE PROTEASE INHIBITORS

| Digoxin Level in Sample (20 µL) After Spiking Sera | aca™ Analyzer Response (m.A.U./min at 405 nm) | Number of Sera | Packs |
|---|---|---|---|
| 0 ƒg/mL | 258.8 ± 4 | 5 | 10 |
| 5 | 308.4 ± 4.9 | 5 | 10 |
| 10 | 328.6 ± 6.8 | 5 | 10 |

F. Cascade-amplified Immunoassay for Digoxin in Serum Using an Ion-Exchange Column to Eliminate Protease Inhibitors

Preparation of monovalent Fab fragments from antidigoxin IgG

50 mg goat anti-digoxin IgG (used in D) was diluted to 2.5 mL in MES buffer, pH 6.5 (20 mM final concentration) containing 10 mM cysteine, 2mM EDTA, and 0.5 mg papain (from Sigma, 2X recrystallized). After 18 hours at 22°C, papain and low-molecular-weight material were removed by passage of the reaction mixture over a Sephacyl S-200 (Pharmacia) column (1.4 x 48 cm) equilibrated and eluted with 5 mM tris HCl, 50 mM NaCl, 0.1% azide, pH 7.5 at 22°C. As monitored by absorbance at 280 nm, residual intact IgG eluted first and was separated from fragments (Fab, Fc) in the subsequent major peak. Fractions from the major peak were pooled (22 mg protein) and diluted to 0.65 mg/mL antibody protein using 1 mg/mL HSA.

Large-scale ultrapurification of Enteropeptidase in three chromatographic steps

For cascade-amplified trypsinogen-activation immunoassays, minimal nonspecific background reaction requires ultrapurified enteropeptidase. This was prepared at 22°C using a combination of column chromatographic steps.

Crude enteropeptidase (Miles Laboratories, 2.5 gm protein, 9625 aca$^{TM}$ analyzer units) was dissolved in deionized water to a final volume of 25 mL. After centrifugation at 18,000 xg for five minutes, the clarified brown supernatant was loaded on a 1.8 L Sephacryl S-300 column (5 x 92 cm) equilibrated with column buffer (5 mM Tris HCl, 50 mM NaCl, 0.1% azide, pH 7.5 at 22°). 7.5 mL fractions were collected at a flow rate of 4.9 mL per minute.

Enteropeptidase activity (assayed as in Example IIB) eluted in the leading portion of the first, minor protein peak. A second, larger protein peak preceded the elution of nonspecific esterase contaminants, which were completely resolved from enteropeptidase. Enteropeptidase fractions were pooled (330 ml, A280 = 1.55) and loaded on a 50 mL (2.5 x 10 cm) DEAE Sephacel column equilibrated with column buffer. Elution was carried out with a 50 - 400 mm linear NaCl gradient. Enteropeptidase fractions eluted slightly after the peak brown fractions; the former were pooled (59.5 mL, A280 = 5.35) and 50 mL were loaded on a 490 mL Sepharose 6BCL column, (Pharmacia, 2.5 x 100 cm) equilibrated with column buffer. The single major protein peak (160 mL, A280 = 1.2) contained all of the enteropeptidase activity (1 aca$^{TM}$ unit per 50 µL). Nonspecific chymotrypsinogen activation with 2 aca$^{TM}$ units of enteropeptidase was 120 mA/min (assayed as in B), compared to 525 mA/min with 0.25 aca$^{TM}$ units purified only by Sephacryl S-300.

### Preparation of Ion Exchange
### Column for digoxin immunoassay

DEAE-Sepharose (Pharmacia, 400 mL) was washed under vacuum in a coarse sintered-glass Buchner funnel with 2000 mL of 1.5M NaCl, followed by 2000 mL deionized water, and finally 2000 mL of 50 mm Tris HCl buffer (pH 8.0 at 22°C). A slurry of washed DEAE-Sepharose was loaded by vacuum into columns (5.5 mm x 7.5 cm) for use in analytical test packs.

### Cascade-amplified Immunoassay
### for Digoxin In Serum

Six normal sera with and without added digoxin (50, 100, 200, 300 μg/mL final concentration, diluted from a 300 μg/mL stock solution in ethanol) were assayed in the aca$^{TM}$ analyzer after fully automated elution through an ion exchange column contained in the pack header. A 150 μL sample was injected into the pack header column and eluted with 2.60 mL $H_2O$ at 17 μL/second. An additional 2.25 mL of concentrated diluent was injected directly into the packs to bring the final buffer concentration to that of standard pack diluent.

Trypsinogen-digoxin conjugate (0.25 μg in 50 μL 5 mM formate, 1 mg/mL HSA), enteropeptidase (1 aca$^{TM}$ unit, purified as described above), and anti-digoxin Fab (32 μg protein) were released at breaker/mixer I. The chromogenic chymotrypsin substrate S-2586 (0.71 mg in 100 μL 0.1% azide) was released at breaker/mixer II.

Response is shown in Table V. Of six sera tested, five fit a single standard curve, while one was still highly inhibited and yielded an off-scale response. A smaller sample size or higher-capacity column would probably eliminate inhibitors for most or all samples. Other experiments show that DEAE-affigel Blue (Biorad) eliminates serum

50

inhibitors for 500 µL samples, but requires a somewhat slower elution rate and larger elution volume than DEAE-sepharose.

## Table V

### CASCADE-AMPLIFIED IMMUNOASSAY FOR DIGOXIN IN SERUM USING DEAE-SEPHAROSE TO ELIMINATE PROTEASE INHIBITORS

| Digoxin Level in Sample (150 µL) After Spiking Sera | aca™ Analyzer Response (m.A.U./min at 405 nm) | Number of Sera Packs | |
|---|---|---|---|
| 0 ng/mL | 609 ± 14 | 5 | 9 |
| 50 | 668 ± 15 | 5 | 9 |
| 100 | 702 ± 9 | 5 | 8 |
| 200 | 741 ± 6 | 3 | 5 |

## EXAMPLE IV

### COMPETITIVE HETEROGENEOUS IMMUNOASSAY USING A ZYMOGEN-LABELED ANALYTE

The following is a description of the best mode contemplated at the present time for carrying out a competitive heterogeneous immunoassay using a zymogen-labled analyte:

Sample, usually 50 µL, suspected of containing analyte is mixed with a stoichiometric amount of trypsinogen analyte conjugate (2-4 analyte molecules per trypsinogen).

The resultant solution is then passed over a 2 mm x 10 mm column of immobilized anti-analyte antibody, the number of antibody binding sites being just sufficient to bind all of the labeled analyte were no analyte present in sample. The flow rate is maintained at 1.0 mL/min and the column is washed with 5.0 mL of diluent containing 10 aca$^{TM}$ analyzer units of enteropeptidase (purified as described in Example II). Finally, 6 mL of diluent containing 0.5 mg of trypsin substrate (S-2444) is flushed through the column and the final 5 mL is collected. An end-point absorbance measurement is then made.

For greater sensitivity, the enteropeptidase wash step is followed by a wash with 5 mL diluent containing 1 µM chymotrypsinogen (purified as described in Example III), made just before use by adding .05 mL of 1 mM chymotrypsinogen (25 mg/mL) to 4.95 mL diluent. This wash is collected as a single fraction and assayed after 5 minutes at 37°C by addition of 0.7 mg of S-2586 to produce a cascade amplified enzyme rate.

It is believed that no special steps need to be taken to remove potential interferences, since these will be substantially eliminated during the enteropeptidase wash of the column.

## EXAMPLE V

### COMPETITIVE HETEROGENEOUS IMMUNOASSAY USING AN ACTIVATOR-LABELED ANALYTE

The assay is performed exactly as described in Example IV above, except analyte or analyte-analog is labeled with enteropeptidase instead of trypsinogen and the column is washed with trypsinogen solution and assayed as described in Example VII.

The amount of signal produce at 405 nm will be inversely proportional to the amount of analyte initially present in the sample.

## EXAMPLE VI

### NONCOMPETITIVE HETEROGENEOUS IA USING AN ACTIVATOR-LABELED ANTI-ANALYTE ANTIBODY

The following is a description of the best mode contemplated at the present time for carrying out a heterogeneous, noncompetitive immunoassay according to the method of this invention:

An enteropeptidase-labeled anti-analyte antibody (as an affinity-purified IgG fraction) is pre-incubated with a known volume, usually 50 μL, of sample suspected of containing analyte. Labeled antibody is usually present in at least a fifty-fold molar excess over analyte. After a 5 to 60 minute incubation at room temperature, the solution is passed over a column, preferably of dimensions 2 mm x 10 mm, of analyte or analyte analog immobilized on a packing material such as Sephadex G-10 at a flow rate of 1.0 mL/min. The amount of immobilized analyte or analyte analog should be at least sufficient to bind all the free labeled antibody present in solution. The column is then washed with 2.75 mL of diluent of (50 mM tris buffer, pH 8.9 at 23°C, containing 5 mM $CaCl_2$). A total volume of 2.8 mL is collected and assayed at 37°C in 5 mL final volume by adding diluent, trypsinogen (to 1 μM), plus chymotrypsin (to

0123265

100 μM) as a decoy protease to inactivate potential interferents in the sample. After a brief incubation (typically 3.5 minutes) the trypsin substrate S-2444 (0.5 mg) is added.

For low level analytes, a cascade is employed in which 10 μM chymotrypsinogen is added with the trypsinogen and 0.7 mg of the chymotrypsin-specific substrate, S-2586, is added in place of S-2444 substrate. In this mode, chymotrypsin cannot be used as a decoy protease. Instead, sample is passed over a 2 mm x 10 mm column of DEAE-Sepharose prior to preincubation with labeled anti-analyte and 500 μL of eluate collected and assayed as above.

The amount of signal produced at 405 nm will be directly proportional to the amount of analyte initially present in the sample.

## EXAMPLE VII

### SANDWICH IMMUNOASSAY

The following is a description of the best mode contemplated at the present time for carrying out a heterogeneous sandwich-type immunoassay according to the method of this invention:

Sample, usually 50 μL, suspected of containing analyte is passed over a column (2 mm x 10 mm) of immobilized anti-analyte (present in approximately 100-fold molar excess relative to analyte) at a flow rate of 1.0 ml/min. Then enteropeptidase labeled anti-analyte (as an IgG fraction) present in 100-fold molar excess over analyte, is passed through the same column containing immunochemically bound analyte. The labeled anti-analyte can be the same antibody as the immobilized antibody or it can be different, most often, it will be different. The column is washed at 2 ml per minute with 10.0 mL of diluent, then with 5 mL of trypsinogen in diluent, prepared just before use by mixing 0.5 mL of 10 µM trypsinogen (in 5 mM formic acid, 1 mg/mL HSA, 10 µM benzamidine) with 4.5 mL diluent. A 5 mL fraction is collected, and after a 5 minute incubation at 37°C, trypsin substrate is added (0.5 mg S-2444) and absorbance is measured.

For low level analytes, a cascade is employed in which the trypsinogen wash fraction is made 10 µM in chymotrypsinogen, and 0.7 mg of the chymotrypsin-specific substrate, S-2586, is added in place of S-2444 substrate. It is believed that no special steps need to be taken to remove potential interferents, since these should be substantially eliminated during the column washing step prior to trypsinogen addition.

The amount of signal produced at 405 nm is directly proportional to the amount of analyte initially present in the sample.

<div align="center">

EXAMPLE VIII

IMMUNODISPLACEMENT ASSAY

</div>

The following is a description of the best mode contemplated at the present time for carrying out an immuno displacement assay according to the method of this invention:

A known volume of sample, usually 5 µL to 500 µL, suspected of containing analyte is passed through a column (2 mm x 10 mm) containing immobilized analyte analog, presaturated with enteropeptidase-labeled monovalent anti-analyte antibody. The analyte-analog and hence the labeled anti-analyte antibody are present in 10-fold molar excess relative to analyte. The column is washed with 50 mM Tris buffer, pH 8.9 (23 C), containing 5 mM $CaCl_2$, at a flow rate of 0.5 mL/min. A total volume of 5 mL is collected and assayed at 37°C as described in Example VI above.

The amount of signal produced at 405 nm is directly proportional to the amount of analyte initially present in the sample.

It will be apparent that the instant specification and examples are set forth by way of illustration and not limitation, and that various modifications and changes may be made without departing from the spirit and scope of the present invention.

WHAT IS CLAIMED IS:

1. A conjugate of an analyte and a labeling substance selected from the group consisting of zymogen, enzymatic activator, and nonenzymatic activator wherein at least one analyte derivative molecule is attached per molecule of labeling substance.

2. The conjugate of claim 1 wherein the labeling substance is a zymogen.

3. The conjugate of claim 2 wherein the zymogen is trypsinogen.

4. The conjugate of claim 1 wherein the attachment of analyte derivative is through an amino acid residue selected from the group consisting of lysine, serine, threonine, tyrosine, aspartate, glutamate, cysteine, hydroxyproline, and C-terminal or N-terminal amino acids.

5. The conjugate of claim 1 wherein the analyte derivative is a drug, metabolite, hormone, steroid, pesticide, environmental pollutant, food toxin, vitamin, peptide, protein, microbial surface marker or cancer cell marker.

6. The conjugate of claim 1 wherein the analyte derivative is a drug selected from the group consisting of digoxin, theophylline, and gentamicin.

7. A method for determining the amount of an analyte in liquid medium comprising the steps of:

    A) forming a reaction system by contacting said liquid medium with

        (1) conjugate of an analyte and a zymogen;

        (2) analyte-specific binding agent;

        (3) activator specific for the conversion of said conjugate of an analyte and a zymogen to zymogen-derived-enzyme;

      (4)    substrate reagent convertible by said zymogen-derived-enzyme to a detectable marker material

in proportions such that the conversion of uncombined conjugate of an analyte and a zymogen by said activator to said zymogen-derived-enzyme results in the production of said marker material; and

      B) measuring the amount of said marker material produced which is related to the amount of analyte initially present in said liquid medium.

8. The method of claim 7 wherein the binding agent is an analyte specific antibody.

9. The method of claim 7 wherein the analyte is a drug, metabolite, hormone, steroid, pesticide, environmental pollutant, food toxin, vitamin, peptide, protein, microbial surface marker, or cancer cell marker.

10. The method of claim 9 wherein the analyte is a drug.

11. The method of claim 10 wherein the drug is selected from the group consisting of digoxin, theophylline, and gentamicin.

12. The method of claim 7 further comprising incorporating in said reaction system zymogens which interact in a coupled zymogen activation cascade initiated by said zymogen-derived-enzyme thereby producing a final enzyme product which converts said substrate reagent to said marker material.

13. The method of claim 12 wherein the binding agent is an analyte specific antibody.

14. The method of claim 12 wherein the analyte is a drug, metabolite, hormone, steroid, pesticide, environmental pollutant, food toxin, vitamin, peptide, protein, microbial surface marker, or cancer cell marker.

15. The method of claim 14 wherein the analyte is a drug.

16. The method of claim 15 wherein the drug is selected from the group consisting of digoxin, theophylline, and gentamicin.

17. The method of claim 7 wherein the conjugate of an analyte and a zymogen is selected from the group consisting of digoxin-trypsinogen, theophylline-trypsinogen, and gentamicin-trypsinogen.

18. The method of claim 7 wherein the activator is enteropeptidase.

19. The method of claim 12 wherein the zymogen reaction cascade consists of the conversion of chymotrypsinogen to chymotrypsin.

20. A method for determining the amount of an analyte in liquid medium comprising the steps of:

    A) forming a reaction system by contacting said liquid medium with

       (1) conjugate of an analyte and an enzyme;

       (2) analyte-specific binding agent;

       (3) zymogens which interact via a coupled zymogen activation cascade initiated by said conjugate of an analyte and an enzyme to produce a final cascade enzyme product; and

       (4) substrate reagent convertible by said final cascade enzyme product of said activation cascade to a detectable marker material

in proportions such that said conjugate of an analyte and an enzyme is substantially inhibited by analyte-specific binding agent in the absence of analyte;

B) measuring the amount of said marker material produced which is related to the amount of analyte initially present in said liquid medium.

21. The method of Claim 20 wherein the enzyme of said conjugate of an analyte and an enzyme is trypsin.

22. The method of Claim 20 wherein the enzyme of said conjugate of an analyte and an enzyme is enteropeptidase.

23. A method for determining the amount of digoxin in liquid medium comprising the steps of:

A) forming a reaction system by contacting said liquid medium with

(1) conjugate of digoxin and trypsinogen;

(2) digoxin-specific antibody;

(3) enteropeptidase specific for the conversion of said conjugate of digoxin and trypsinogen to digoxin-trypsin conjugate;

(4) chymotrypsinogen which is convertible to chymotrypsin by said digoxin-trypsin conjugate; and

(5) substrate reagent convertible by said chymotrypsin to a detectable marker material in proportions such that the conversion of uncombined conjugate of digoxin and trypsinogen by said enteropeptidase to said digoxin-trypsin conjugate results in the production of said marker material; and

B) measuring the amount of said marker material produced which is related to the amount of digoxin initially present in said liquid medium.

24. A method for determining the amount of an analyte in liquid medium comprising the steps of:

A) forming a reaction system by contacting said liquid medium with

    (1) conjugate of an analyte and a labeling substance selected from the group consisting of zymogen, enzymatic activator, and nonenzymatic activator;

    (2) insolubilized binding agent;

B) allowing the immunochemical reaction to proceed to form free and bound conjugate;

C) separating said conjugate bound to said insolubilized binding agent from said conjugate remaining free in solution;

D) contacting and reacting said conjugate of an analyte and a labeling substance free in solution with a corresponding bioreactive substance selected from the group consisting of activator and zymogen, and with substrate reagent convertible by the resulting zymogen-derived-enzyme to a detectable marker material; and

E) measuring the amount of said marker material produced which is related to the amount of analyte initially present in said liquid medium;

with the proviso that when said labeling substance is zymogen, said corresponding bioreactive substance is activator; when said labeling substance is enzymatic activator, said corresponding bioreactive substance is zymogen; and when said labeling substance is nonenzymatic activator, said corresponding bioreactive substance is zymogen.

25. The method of claim 24 wherein the binding agent is an analyte specific antibody.

26. The method of claim 24 wherein the labeling substance is a zymogen.

27. The method of Claim 26 wherein the zymogen is trypsinogen.

28. The method of claim 24 wherein the labeling substance is an enzymatic activator of a zymogen.

29. The method of claim 28 wherein the enzymatic activator is enteropeptidase.

30. The method of claim 28 wherein the enzymatic activator is trypsin.

31. The method of claim 24 wherein the labeling substance is a nonenzymatic activator of a zymogen.

32. The method of claim 31 wherein the nonenzymatic activator is streptokinase.

33. The method of claim 24 further comprising incorporation in step D) zymogens which interact in a coupled zymogen activation cascade initiated by said zymogen-derived-enzyme thereby producing a final enzyme product which converts said substrate reagent to said marker material.

34. A conjugate of a specific binding agent and a labeling substance selected from the group consisting of zymogen, enzymatic activator and nonenzymatic activator wherein at least one molecule of said binding agent is attached per molecule of labeling substance.

35. The conjugate of Claim 34 wherein the labeling substance is a zymogen.

36. The conjugate of claim 35 wherein the zymogen is trypsinogen.

37. The conjugate of claim 34 wherein the labeling substance is an enzymatic activator capable of activating a zymogen.

38. The conjugate of claim 37 wherein the enzymatic activator is enteropeptidase.

39. The conjugate of claim 37 wherein the enzymatic activator is trypsin.

40. The conjugate of claim 34 wherein the labeling substance is a nonenzymatic activator capable of activating a zymogen.

41. The conjugate of claim 40 wherein the nonenzymatic activator is streptokinase.

42. A method for determining the amount of an analyte in liquid medium comprising the steps of:

    A) forming a reaction system by contacting said liquid medium with

       (1) conjugate of a specific binding agent and a labeling substance selected from the group consisting of zymogen, enzymatic activator, and nonenzymatic activator;

       (2) insolubilized analyte;

    B) allowing the immunochemical reaction to proceed to form free and bound conjugate;

    C) separating said conjugate bound to said insolubilized analyte from said conjugate remaining free in solution;

D) contacting and reacting said conjugate of a specific binding agent and a labeling substance free in solution with a corresponding bioreactive substance selected from the group consisting of activator and zymogen, and with substrate reagent convertible by the resulting zymogen-derived-enzyme to a detectable marker material; and

E) measuring the amount of said marker material produced which is related to the amount of analyte initially present in said liquid medium; with the proviso that when said labeling substance is zymogen, said corresponding bioreactive substance is activator; when said labeling substance is enzymatic activator, said corresponding bioreactive substance is zymogen; and when said labeling substance is nonenzymatic activator, said corresponding bioreactive substance is zymogen.

43. The method of claim 42 wherein the binding agent is an analyte-specific antibody.

44. The method of claim 42 wherein the labeling substance is a zymogen.

45. The method of claim 44 wherein the zymogen is trypsinogen.

46. The method of claim 42 wherein the labeling substance is an enzymatic activator of a zymogen.

47. The method of claim 46 wherein the enzymatic activator is enteropeptidase.

48. The method of claim 46 wherein the enzymatic activator is trypsin.

49. The method of claim 42 wherein the labeling substance is a nonenzymatic activator of a zymogen.

50. The method of claim 49 wherein the nonenzymatic activator is streptokinase.

51. The method of claim 42 further comprising incorporating in step D) zymogens which interact in a coupled zymogen activation cascade initiated by said zymogen-derived-enzyme thereby producing a final enzyme product which converts said substrate reagent to said marker material.

52. A method for determining the amount of an analyte in liquid medium comprising the steps of:

A) forming a reaction matrix by contacting said liquid medium with insolubilized binding agent to produce bound insolubilized analyte;

B) contacting said reaction matrix formed in step A) with conjugate of a specific binding agent and a labeling substance selected from the group consisting of zymogen, enzymatic activator and nonenzymatic activator;

C) rinsing said reaction matrix of step B) with solvent to remove unbound reagent;

D) contacting and reacting said reaction matrix of step C) with a corresponding bioreactive substance selected from the group consisting of activator and zymogen, and with substrate reagent convertible by the resulting zymogen-derived-enzyme to a detectable marker material; and

E) measuring the amount of said marker material produced which is related to the amount of analyte initially present in said liquid medium; with the proviso that when said labeling substance is zymogen, said corresponding bioreactive substance is activator; when said labeling substance is enzymatic activator, said corresponding bioreactive substance is zymogen; and when said labeling substance is nonenzymatic activator, said corresponding bioreactive substance is zymogen.

53. The method of claim 52 wherein said labeling substance is enteropeptidase and said corresponding bioreactive substance is trypsinogen.

54. The method of claim 52 further comprising incorporating in step D) zymogens which interact in a coupled zymogen activation cascade initiated by said zymogen-derived-enzyme thereby producing a final enzyme product which converts said substrate reagent to said marker material.

0123265

Fig. 1

TRYPSINOGEN ACTIVATION ASSAY FOR GENTAMICIN

0123265

Fig. 2

TRYPSINOGEN ACTIVATION ASSAY FOR THEOPHYLLINE

0123265

Fig. 3

TRYPSIN ASSAY CURVE

Fig. 4

CASCADE AMPLIFIED DIGOXIN ASSAY

EP 84104371.4

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ²) |
|---|---|---|---|
| D,A | US - A - 4 434 236 (J.W. FREYTAG) <br> * Abstract * <br> -- | 1,5,7, 9,11 | G 01 N 33/54 <br><br> G 01 N 33/58 <br><br> C 12 Q 1/38 |
| D,A | US - A - 4 238 565 (W.E. HORNBY et al.) <br> * Abstract * <br> -- | 1,5,7 | |
| D,A | US - A - 3 654 090 (A.H.W.M. SCHUURS AND B.K. VAN WEEMEN) <br> * Abstract * <br> ---- | 1,5,7 | |

| TECHNICAL FIELDS SEARCHED (Int. Cl. ³) |
|---|
| G 01 N 33/00 <br><br> C 12 Q <br><br> C 07 G |

The present search report has been drawn up for all claims

| Place of search | Date of completion of the search | Examiner |
|---|---|---|
| VIENNA | 20-07-1984 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82